# EUROPÄISCHE PATENTSCHRIFT

(11) **EP 0 956 282 B1**
(45) Veröffentlichungstag und Bekanntmachung des Hinweises auf die Patenterteilung: **17.04.2002**
(21) Anmeldenummer: 98936404.7
(22) Anmeldetag: 01.07.1998
(51) Int. Cl.: C07C 313/04, C08F 2/22, C09C 1/42, C09D 9/00, D06L 3/10, D21C 9/10

(54) **SULFINSÄUREDERIVATE UND DEREN HERSTELLUNG UND VERWENDUNG**
SULPHINIC ACID DERIVATIVES, METHOD FOR PRODUCING THEM, AND THEIR USE
DERIVES D'ACIDE SULFINIQUE, LEUR PRODUCTION ET LEUR UTILISATION

(30) Priorität: 02.10.1997 DE 19743759
(43) Veröffentlichungstag der Anmeldung: 17.11.1999
(73) Patentinhaber: L. Brüggemann KG, 74076 Heilbronn (DE)
(72) Erfinder: BERGHOFER, Josef, D-74080 Heilbronn (DE); ROTHMANN, Harry, D-74915 Daisbach (DE)
(74) Vertreter: Kinzebach, Werner, Dr.
(86) Internationale Anmeldenummer: EP9804055
(87) Internationale Veröffentlichungsnummer: WO9918067

(56) Entgegenhaltungen:
- DE-A- 2 745 274
- DE-A- 19 510 278
- DE-B- 1 195 742
- DE-B- 1 240 035
- DE-C- 841 912
- GB-A- 758 150
- M. MULLIEZ, ET AL.: "Synthèse d'alpha-hydroxysulfinates" TETRAHEDRON, Bd. 49, Nr. 12, März 1993, Seiten 2469-2476, XP002029797 OXFORD, GB
- PATENT ABSTRACTS OF JAPAN vol. 009, no. 180 (C-293), 25. Juli 1985 & JP 60 049068 A (MITSUBISHI ENPITSU, ET AL.), 18. März 1985
- J.R. NOOI, ET AL.: "Reactions of photoexcited SO2; preparation of alpha-substituted alkanesulphinic acids" TETRAHEDRON LETTERS, Nr. 29, Juni 1970, Seiten 2531-2534, XP002081713 OXFORD, GB
- CHEMICAL ABSTRACTS, vol. 96, no. 8, 22. Februar 1982 Columbus, Ohio, US; abstract no. 54156f, Seite 113; XP002081714 & SU 870 538 B (ALL-UNION SCI.-IND. ENTERPRISES OF THE CELLULOSE-PAPER INDUSTRY, PERM) 7. Oktober 1981

## Beschreibung

Die Erfindung betrifft Sulfinsäurederivate und deren Herstellung und Verwendungen in verschiedenen Einsatzgebieten.

Bekanntlich handelt es sich bei der Sulfinsäure, H₂SO₂, um eines der stärksten bekannten Reduktionsmittel. Die freie Sulfinsäure ist nicht stabil. Dementsprechend kommt sie nur in Form ihrer stabilen und entsprechend handhabbaren Derivate in den Handel.

Bis heute haben folgende Sulfinsäurederivate wirtschaftliche Bedeutung erlangt:
1. Natriumdithionit (Faserbleiche in der Papierherstellung, Küpenfärbung und Textilbleiche, Mineralbleiche, Schwermetallreduktion in Industrieabwässern)
2. Natriumformaldehydsulfoxylat-Dihydrat (Textilätzdruck, Textilbleiche, Redox-Cokatalysator in der Emulsionspolymerisation, Schwermetallreduktion, Pharmazie)
3. Formamidinsulfinsäure (Faserbleiche in der Papierherstellung, Textilbleiche)
4. Zinkformaldehydsulfoxylat (Textildruck und Textilbleiche)

Alle oben genannten Sulfinsäurederivate werden in Form wäßriger Lösungen oder Dispersionen angewandt. In wäßrigen Medien sind das Natriumdithionit und das Alkaliformamidinsulfinat - die freie Formamidinsulfinsäure ist sehr schlecht wasserlöslich und besitzt in ihrer Säureform nur eine sehr geringe Reduktionswirkung - nur kurzzeitig stabil. Dafür zeigen sie bereits bei Raumtemperatur ein hervorragendes Reduktionsvermögen bzw. eine hervorragende Bleichwirkung auf Faserstoffe. Wässrige Zubereitungen des Natriumformaldehydsulfoxylates und des Zinkformaldehydsulfoxylates sind bei Raumtemperatur monatelang stabil. Dafür entfalten beide Formaldehydsulfoxylate ihre eigentliche Reduktionswirkung erst bei Temperaturen oberhalb 90° Celsius. In stark alkalischen oder sauren Medien oder in Gegenwart entsprechend starker Oxidationsmittel reduzieren beide Formaldehydsulfoxylate natürlich auch bei niedrigeren Temperaturen als 90°C. Diese besondere Eigenschaft der Formaldehydsulfoxylate, nämlich bei Temperaturen zwischen 5°C und 90°C eine sehr gleichmäßige und gut kontrollierbare Reduktionswirkung zu entfalten, macht man sich in der radikalisch initiierten Emulsionspolymerisation zu Nutze. Dabei werden die Formaldehydsulfoxylate in verschiedenen Emulsionspolymerisationssystemen eingesetzt. Im Fall der Kalt-SBR (Styrol-Butadien-Kautschuk)-Erzeugung wird die Polymerisation mit organischen Peroxiden initiiert. Bei der niedrigen Polymerisationstemperatur von ca. 5°C zerfallen die organischen Peroxide jedoch nicht in die benötigten Radikale. Die Peroxidspaltung muß durch katalytische Mengen von Eisen(II)salzen eingeleitet werden. Das Eisen in der Oxidationsstufe zwei wird dabei in die Oxidationsstufe drei überführt, in welcher es für die Peroxidspaltung nicht mehr geeignet ist. Mit Hilfe des Formaldehydsulfoxylates werden die Eisen(III)-Ionen wieder zu Eisen(II)-Ionen reduziert - die Peroxidspaltung und die Radikalinitiierung laufen weiter. In anderen Emulsionspolymerisationssystemen werden Peroxidverbindungen, wie Wasserstoffperoxid oder Peroxodisulfat, als Radikalbildner verwendet. Um die Radikalbildungsrate zu erhöhen, setzt man wiederum Reduktionsmittel ein. Als Beispiel seien genannt Formaldehydsulfoxylate, Bisulfite, Ascorbinsäure, Isoascorbinsäure und Natriumerythrobat. Die Formaldehydsulfoxylate, insbesondere das Natriumformaldehydsulfoxylat, haben sich als besonders effektive und preisgünstige Reduktionsmittel bewährt. Während des Reduktionsvorganges spalten die Formaldehydsulfoxylate jedoch Formaldehyd ab. Kunststoffe oder Polymerdispersionen, die keinen Formaldehyd enthalten dürfen, werden entweder mit Bisulfiten, Ascorbinsäure, Isoascorbinsäure oder Natriumerythrobat polymerisiert. Da es sich bei den formaldehydfreien Reduktionsmitteln um schwächere Reduktionsmittel handelt, muß man im Vergleich zu Formaldehydsulfoxylaten den Nachteil der unvollständigeren Polymerisation in Kauf nehmen. Außerdem führt der Einsatz der Ascorbinsäure, Isoascorbinsäure und des Natriumerythrobates zu einer unerwünschten Vergilbung des Polymerisates.

Die DE 1240035A beschreibt ein Verfahren zur kontinuierlichen Färbung von Bahnen aus Fasermaterial mit Küpen- oder Schwefelfarbstoffen, wobei als Reduktionsmittel Verbindungen der aligemeinen Formel 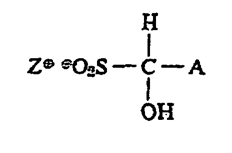 verwendet werden, worin A für Phenyl steht, das ggf. durch ein oder mehrere Alkyl-, Hydroxyl-, Alkoxyl-, Amino-, Halogen- oder Sulfogruppen substituiert ist und Z^{⊕} ein Alkali- oder Amoniumkation bedeutet.

Die DE 19510278 A beschreibt ein Bleichmittel zur Aufhellung von Sekundärfaserstoffen aus Altpapier, das ein Dithionitsalz und ein wasserlösliches Metallsalz einer Hydroxialkansulfinsäure der Formel 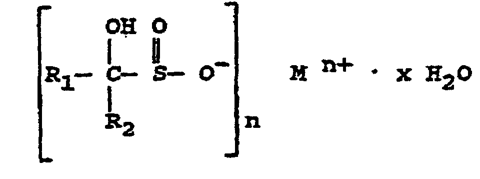 enthält, wobei R₁ und R₂ für ein Wasserstoffatom, eine Alkylgruppe mit weniger als fünf Kohlenstoffatomen oder einen Phenylrest stehen, x für ein Zahl von 0 bis 6 steht, n für 1 oder 2 steht und M für ein Alkali- oder Erdalkalimetallatom steht.

DE 841912 A beschreibt ein Verfahren zur Herstellung von oxyalkyl- oder oxyarylsulfinsauren Salzen.

Die DE 1195742 A beschreibt ein Verfahren zur Herstellung von Ketosulfinsäuren der Formel 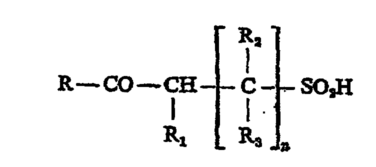 worin R ein aliphatischer, zykloaliphatischer oder aromatischer Rest ist, R₁ insbesondere für COOR, CN, SO₂R oder COR steht, R₂ und R₃ für H, Alkyl oder Aryl stehen und n für 1 bis 4 steht.

Der vorliegenden Erfindung liegt die Aufgabe zugrunde, neue Sulfinsäurederivate zur Verfügung zu stellen, deren chemische Eigenschaften möglichst ähnlich denen der Formaldehydsulfoxylate sind, die aber während und nach der Anwendung kein Formaldehyd abspalten.

Überraschenderweise wurde nun gefunden, daß diese Aufgabe gelöst wird durch Sulfinsäurederivate der unten näher bezeichneten Art.

Gegenstand der vorliegenden Erfindung sind daher Sulfinsäureverbindungen der allgemeinen Formel (I) : 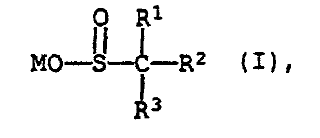 worin
- M: für ein Wasserstoffatom, ein Ammoniumion, ein einwertiges Metallion oder ein,Äquivalent eines zweiwertigen Metallions der Gruppen Ia, IIa, IIb, IVa oder VIIIb des Periodensystems steht;
- R¹: für OH oder NR⁴R⁵ steht, wobei R⁴ und R⁵ unabhängig voneinander für H oder C₁-C₆-Alkyl stehen;
- R²: für H oder eine Alkyl-, Alkenyl-, Cycloalkyl- oder Arylgruppe steht, wobei diese Gruppe 1, 2 oder 3 Substituenten aufweisen kann, die unabhängig voneinander ausgewählt sind unter C₁-C₆-Alkyl, OH, O-C₁-C₆-Alkyl, Halogen und CF₃; und
- R³: für COOM, SO₃M, COR⁴, CONR⁴R⁵ oder COOR⁴ steht, wobei M, R⁴ und R⁵ die oben angegebenen Bedeutungen besitzen,
und die Salze davon.

Im Rahmen der vorliegenden Erfindung besitzen die nachfolgend aufgeführten Ausdrücke folgende Bedeutungen:
Alkyl steht für geradkettige oder verzweigte Alkylgruppen, die vorzugsweise 1 - 6, insbesondere 1 - 4 Kohlenstoffatome aufweisen. Beispiele für Alkylgruppen sind Methyl, Ethyl, n-Propyl, Isopropyl, n-Butyl, t-Butyl, n-Hexyl etc.
Entsprechendes gilt für die Alkylgruppen in O-Alkyl.
Alkenyl steht für geradkettige oder verzweigte Alkenylgruppen, die vorzugsweise 3 - 8 Kohlenstoffatome, insbesondere 3 - 6 Kohlenstoffatome aufweisen. Eine bevorzugte Alkenylgruppe ist die Allylgruppe.
Cycloalkyl steht insbesondere für C₃-C₆-Cycloalkyl, wobei Cyclopentyl und Cyclohexyl besonders bevorzugt sind.
Aryl (auch in Aralkyl) steht vorzugsweise für Phenyl oder Naphthyl.

Wenn der Arylrest für eine Phenylgruppe steht und substituiert ist, so weist er vorzugsweise zwei Substituenten auf. Diese sind insbesondere in 2- und/oder 4-Stellung vorhanden.

Halogen steht für F, Cl, Br und I, vorzugsweise für Cl und Br.

M steht vorzugsweise für ein Ammonium-, Alkalimetall- oder ein Äquivalent eines Erdalkalimetall- oder Zinkions. Geeignete Alkalimetallionen sind insbesondere Natrium- und Kaliumionen, geeignete Erdalkalimetallionen sind vor allem Magnesium- und Calciumionen.

R¹ steht vorzugsweise für eine Hydroxy- oder Aminogruppe.

R² steht vorzugsweise für ein Wasserstoffatom oder eine Alkyl- oder Arylgruppe, die wie oben substituiert sein kann. Vorzugsweise weist sie einen oder zwei Hydroxy- und/oder Alkoxysubstituenten auf.

R³ steht vorzugsweise entweder für COOM oder COOR⁴ (M und R⁴ besitzen die oben angegebenen Bedeutungen).

Eine bevorzugte Ausführungsform sind Verbindungen der Formel (I), worin
- M: für ein Alkalimetallion oder ein Äquivalent eines Erdalkalimetalloder Zinkions steht;
- R¹: für eine Hydroxy- oder Aminogruppe steht; R² für H oder Alkyl steht; und
- R³: für COOM oder COOR⁴ steht, wobei M für H, ein Alkalimetallion oder ein Äquivalent eines Erdalkalimetallions steht und R⁴ für C₁-C₆-Alkyl steht.

Die Herstellung der erfindungsgemäßen Verbindungen erfolgt ausgehend von Dithionitsalzen. Zweckmäßigerweise verwendet man ein Salz mit einem Kation, das auch in den Sulfinsäureverbindungen erwünscht ist.

Die 'Herstellung der Verbindungen der Formel I erfolgt durch Umsetzung der Dithionitsalze mit der entsprechenden 1, 2-Dicarbonylverbindung bzw. einem Sulfonsäureäquivalent davon. Als 1,2-Dicarbonylverbindung verwendet man insbesondere Glyoxylsäure oder die entsprechenden Ketoverbindungen sowie deren Ester. Die Umsetzung läßt sich anhand der nachfolgenden Reaktionsgleichung am Beispiel von Natriumdithionit und Glyoxylsäure veranschaulichen:

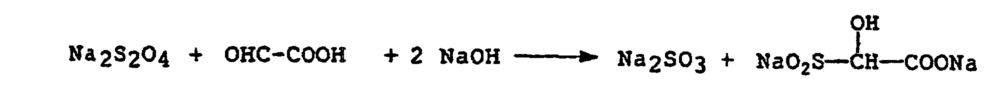

Die Umsetzung erfolgt im allgemeinen in wäßrigem Medium in Anwesenheit einer Base. Das wäßrige Medium kann auch wasserlösliche organische Lösungsmittel umfassen, wie Methanol, Ethanol, Isopropanol etc. Als Basen sind insbesondere Alkali- und Erdalkalimetallhydroxide brauchbar. Im allgemeinen erfolgt die Umsetzung bei Umgebungstemperatur, Erhitzen des Reaktionsgemisches ist in der Regel nicht erforderlich, weil die Reaktion exotherm ist. Das gewünschte Produkt fällt im allgemeinen aus dem Reaktionsgemisch aus oder kann durch Zugabe polarer, wasserlöslicher organischer Lösungsmittel, wie Methanol, Ethanol, Isopropanol, Aceton etc., ausgefällt werden. Das erhaltene Produkt liegt in Form des Salzes vor, das gewünschtenfalls durch Ansäuern oder Behandlung mit einem sauren Ionenaustauscher in die freie Sulfinsäure überführt werden kann.

Außerdem fällt das Produkt im allgemeinen im Gemisch mit dem entsprechenden Metallsulfit an. In vielen Fällen enthält das Gemisch auch die entsprechende Sulfonsäure sowie Kristallwasser. Die erfindungsgemäßen Verbindungen können von den begleitenden Bestandteilen in üblicher Weise abgetrennt werden, beispielsweise durch Umkristallisation aus Wasser oder wäßrigem Alkohol.

Für die Anwendung in der Praxis ist es nicht erforderlich, die begleitenden Bestandteile abzutrennen. Es hat sich vielmehr gezeigt, daß die Wirkung der erfindungsgemäßen Verbindungen durch diese begleitenden Bestandteile sogar noch gesteigert wird. Gegenstand der Erfindung sind daher auch die entsprechenden Gemische mit den erwähnten Bestandteilen. Das Metallsulfit kann dabei in einem Anteil bis zu 40 % und die Sulfonsäure in einem Anteil bis zu 60 % enthalten sein. Der Wassergehalt kann bis zu 30 % betragen.

Die erfindungsgemäßen Verbindungen sind Reduktionsmittel, deren reduzierende Wirkung derjenigen der Formaldehydsulfoxylate vergleichbar ist. Sie besitzen aber den Vorteil, vor, während und nach der Anwendung keinen Formaldehyd abzuspalten. Die erfindungsgemäßen Verbin-dungen finden daher bevorzugt auf solchen Gebieten Anwendung, bei denen die Entwicklung von Formaldehyd unerwünscht ist. Beispielsweise sind sie als Reduktionsmittel im Textildruck, insbesondere im Textilätzdruck, in der Textilbleiche oder der Küpenfärbung, oder als Reduktionsmittel zum Bleichen von Mineralstoffen, wie Kaolin etc. , und Fasern, beispielsweise Zellulosefasern, brauchbar. Vorzugsweise werden sie jedoch als Co-Katalysator in der Emulsionspolymerisation zusammen mit peroxidischen Initiatoren eingesetzt, um die Durchführung der Polymerisation bei niedrigerer Temperatur zu ermöglichen. Gewünschtenfalls können die Sulfinsäuren zu diesem Zweck auch zusammen mit oxidierbaren Metallionen, wie Fe²⁺, Mn²⁺ etc., eingesetzt werden. Zweckmäßigerweise verwendet man diese Metallionen dann als Gegenionen zu den Sulfinsäureverbindungen, d.h. M = Fe²⁺, Mn²⁺ etc.

Zur Anwendung werden die erfindungsgemäßen Verbindungen im allgemeinen zusammen mit üblichen Zusatz- und Hilfsstoffen formuliert. Eine besondere Beschränkung in dieser Hinsicht besteht nicht, es dürfen lediglich keine reduzierbaren Verbindungen herangezogen werden.

Die nachfolgenden Beispiele erläutern die Erfindung, ohne sie zu beschränken. Die in den Beispielen vorhandenen Reinheitsangaben beziehen sich auf das angefallene kristallwasserhaltige Produkt, d.h. die Reinheit ist erheblich höher, wenn der Anteil des Kristallwassers berücksichtigt wird.

### Beispiel 1

### 2-Hydroxy-2-sulfinatoessigsäure, Dinatriumsalz

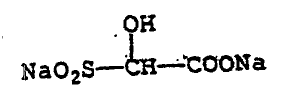

Die Umsetzung von 358 g handelsüblichen Natriumhydrosulfits in 800 ml Wasser mit 268 g 50 %-iger Glyoxylsäure und 285 g 50%iger Natronlauge ergab das 2-Hydroxy-2-sulfinatoessigsäure, Dinatriumsalz mit einer Ausbeute von 95%. Das feste Rohprodukt enthielt 43% Sulfinsäure (ohne Hydratwasser). Durch Kristallisation aus Methanol-Ethanol-Wasser-Gemisch erhielt man das Hydrat der Sulfinsäure in schönen Kristallen. Die Bestimmung der schwefelhaltigen Bestandteile erfolgte durch Iodometrie. Die Sulfinsäure zeigt mit Indanthrenpapier eine Reaktion bei ca. 75°C.

Das IR-Spektrum zeigt folgende Peaks:
3588.57 cm⁻¹ (6.21 %T); 3485.05 cm⁻¹ (1.37 %T); 3339.44 cm⁻¹ (1.75 %T); 2905.13 cm⁻¹ (38.46 %T); 2794.17 cm⁻¹ (42.39 %T); 2189.93 cm⁻¹ (54.06 %T); 1662.54 cm⁻¹ (7.35 %T); 1613.92 cm⁻¹ (0.67 %T); 1417.54 cm⁻¹ (7.34 10 %T); 1388.03 cm⁻¹ (8.65 %T); 1248.31 cm⁻¹ (3.95 %T); 1185.34 cm⁻¹ (30.75 %T); 1153.96 cm⁻¹ (20.95 %T); 1103.16 cm⁻¹ (5.58 %T); 1027.04 cm⁻¹ (2.61 %T); 968.33 cm⁻¹ (1.77 %T); 938.07 cm⁻¹ (26.60 %T); 847.72 cm⁻¹ (23.10 %T); 717.14 cm⁻¹ (10.46 %T); 645.46 cm⁻¹ (14.88 %T); 541.36 cm⁻¹ (9.25 %T); 491.77 cm⁻¹ (11.95 %T); 445.88 cm⁻¹ (19.23 %T).
¹³C-Kernresonanz-Spektrum (63MHz) :
δ (ppm): 93,8 (s) ; 177,7 (s)

### Beispiel 2

### 2-Hydroxy-2-sulfinatoessigsäure, Zinksalz

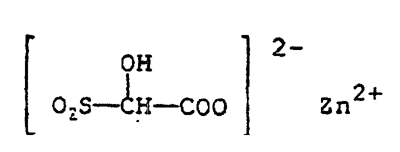

Durch die Umsetzung von 33 g Zn-Staub in wässrigen Medium mit Schwefeldioxid erhielt man Zinkdithionit. Dieses wurde in situ mit 136 g 50%iger Glyoxylsäure umgesetzt. Nach Beendigung der exothermen Reaktion wurden 75 g ZnO zugegeben. Das im Filtrat enthaltene Rohprodukt wurde mit Methanol ausgefällt und setzte sich zusammen aus 20% Sulfin und 48% Sulfonsäure (Iodometrische Bestimmung).

### Beispiel 3

### 2-Hydroxy-2-Sulfinatopropionsäure, Dinatriumsalz

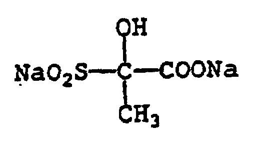

Ausgehend von 89 g handelsüblichem Natriumhydrosulfit in Wasser erhielt man das Rohprodukt durch Umsetzung mit 40 g Brenztraubensäure und ca. 78 g 50%ige Natronlauge. Das Rohprodukt enthielt 40% Sulfinsäure und wurde aus Methanol-Ethanol-Wasser-Gemisch umkristallisiert. Der Gehalt wurde iodometrisch bestimmt. Als Nebenbestandteil enthalten ist das entsprechende Sulfonsäure, Dinatriumsalz.
Die gefundenen IR-spektroskopischen Signale lauten wie folgt:
3484.66 cm⁻¹ (6.25 %T); 2995.53 cm⁻¹ (26.51 %T); 2758.93 cm⁻¹ (32.54 %T); 1592.63 cm⁻¹ (0.62 %T); 1456.02 cm⁻¹ (16.06 %T); 1436.19 cm⁻¹ (17.02 %T); 1397.00 cm⁻¹ (4.77 %T); 1367.01 cm⁻¹ (7.14 %T); 1190.80 cm⁻¹ (2.49 %T); 1038.50 cm⁻¹ (0.70 %T); 981.07 cm⁻¹ (1.42 %T); 943.83 cm⁻¹ (7.90 %T); 857.07 cm⁻¹ (20.25 %T); 804.64 cm⁻¹ (32.86 %T); 790.68 cm⁻¹ (34.62 %T);710.08 cm⁻¹ (30.79 %T); 659.00 cm⁻¹ (11.96 %T); 628.53 cm⁻¹ (9.93 %T); 558.19 cm⁻¹ (26.14 %T); 522.56 cm⁻¹ (16.21 %T);497.03 cm⁻¹ (15.70 %T); 431.34 cm⁻¹ (28.83 %T).

### Beispiel 4

### 2-Hydroxy-2-sulfinatopropionsäureethylester, Natriumsalz

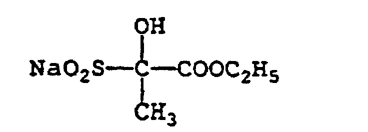

Nach Umsetzung von 90 g handelsüblichem Natriumhydrosulfit in wässriger Lösung mit 60 g Brenztraubensäureethylester und 39 g 50%iger Natronlauge fiel während der exothermen Reaktion das 2-Hydroxy-2-sulfinatopropionsäureethylester, Natriumsalz als Hydrat aus. Das abgetrennte und getrocknete Rohprodukt enthält 79% Sulfinsaure (ohne Kristallwasser gerechnet).
Die Gehaltsbestimmungen erfolgten mittels Iodometrie. Die IR-spektroskopischen Signale lassen sich wie folgt zusammenfassen:
3501.08 cm⁻¹ (12.01 %T); 3328.38 cm⁻¹ (16.14 %T); 3003.23 cm⁻¹ (51.87 %T); 2986.52 cm⁻¹ (45.03 %T); 2940.61cm⁻¹ (54.87 %T);1733.45 cm⁻¹ (7.42 %T); 1663.31 cm⁻¹ (48.05 %T); 1469.00 cm⁻¹ (32.01 %T); 1402.22 cm⁻¹ (42.89 %T); 1367.58 cm⁻¹ (40.81 %T);1298.47 cm⁻¹ (43.49 %T); 1262.97 cm⁻¹ (26.65 %T); 1190.27 cm⁻¹ (10.52 %T); 1105.86 cm⁻¹ (10.94 %T); 1038.98 cm⁻¹ (6.62 %T); 1012.00 cm⁻¹ (30.53 %T); 985.42 cm⁻¹ (9.37 %T); 948.69 cm⁻¹ (28.55 %T); 860.86 cm⁻¹ (56.24 %T); 801.55 cm⁻¹ (61.53 %T);685.30 cm⁻¹ (51.65 %T); 658.49 cm⁻¹ (51.18 %T); 590.17 cm⁻¹ (34.18 %T); 523.55 cm⁻¹ (34.88 %T); 471.89 cm⁻¹ (41.25 %T); 425.61 cm⁻¹ (59.75 %T).

### Beispiel 5

Für den Textilätzdruck auf schwarzem Stoff wurde eine Druckpaste folgender Formulierung ausgewählt.

| Grundrezeptur der Druckpaste: | |
|---|---|
| 434 g | Wasser |
| 100 g | Pottasche |
| 6 g | Verdickungsmittel KL 100 (carboxymethylierte Stärke) |
| 40 g | Lameprint IND8 (Guarether + Stärkeether) |
| 14 g | Glycerin |
| 6 g | Printogen (selbstemulgierbares Mineralöl) |
| 600 g Grundrezeptur | |

Zu dieser Grundrezeptur wurde nun das formaldehydfreie Reduktionsmittel entsprechend Beispiel 1 bzw. Natriumformaldehydsulfoxylat für die Vergleichsmischung gegeben.

| Mischung 1 | Vergleichsmischung |
|---|---|
| 600 g Grundrezeptur | 600 g Grundrezeptur |
| 213 g 2-Hydroxy-2-sulfinatoessigsäure, Dinatriumsalz entsprechend Beispiel 3 | 107 g Natriumformaldehydsulfoxylat |
| (Rohprodukt) | |

Die so hergestellten Mischungen wurden dann auf den schwarzen Stoff nebeneinander aufgebracht und im Trockenschrank getrocknet. Anschließend wurde der Stoff bei 102°C 10 Minuten gedämpft. Hierbei erfolgte die Reduktion des Farbstoffes. Durch gründliches Auswaschen des Stoffes wurden Reste von Verdickungsmittel und anderen Chemikalien entfernt und der ungefärbte Stoff zeigte sich an den Stellen, wo zuvor das Reduktionsmittel aufgetragen wurde.

Es ist ersichtlich, daß der Ätzdruck gut funktioniert hat. Das Auswaschen der Zubereitungen machte keinerlei Probleme. 2-Hydroxy-2-sulfinatoessigsäure, Dinatriumsalz kann somit nach der gängigen Technologie im Textilätzdruck eingesetzt werden. Die Ergebnisse der Ätzdrucke sind in Tabelle 1 zusammengefaßt:

**Tabelle 1:**

| Weißgrad R457 | Mischung 1 | Vergleichsmischung |
|---|---|---|
| 1. Messung | 69,55 | 71,40 |
| 2. Messung | 70,24 | 70,73 |
| 1. Gelbwert | 9,83 | 8,91 |
| 2. Gelbwert | 9,51 | 9,05 |

### Beispiel 6

### Bleichung von Kaolin

Die Ausgangskonzentration des Kaolins betrug 250 g/l. Die Slurry hatte einen pH-Wert von 6, 5. Nachdem die Kaolinsuspension mit einem Rührer 30 Minuten homogenisiert worden war, wurde der pH-Wert mit halbkonzentrierter Schwefelsäure auf 2,5 eingestellt.

Die Zugabe von 2-Hydroxy-2-sulfinatoessigsäure, Dinatriumsalz entsprechend Beispiel 1 und Natriumformaldehydsulfoxylat erfolgte als 10%-ige Lösung und wurde auf den Feststoffgehalt der Kaolinsuspension bezogen (siehe Tabelle 2).

| Reaktionsbedingungen: | |
|---|---|
| Temperatur | Raumtemperatur |
| pH-Wert | 2,5 |
| Umsetzungsdauer | 2 Stunden |

**Tabelle 2:**

| Kaolintyp | Einsatzmenge [%atro] | Ausgangsweiße [%] R 457 | Endweiße [%] R 457 | Farbton R 457 | Sättigung R 457 |
|---|---|---|---|---|---|
| A | 0,45 | | | | |
| | 2-Hydroxy-2-sulfinatoessigsäure, Dinatriumsalz entsprechend Beispiel 3 | 73,4 | 76,8 | 1,51 | 0,46 |
| A | 0,3 | | | | |
| | Natriumformaldehydsulfoxylat | 73,4 | 74,5 | 1,59 | 0,61 |
| B* | 0,3 | | | | |
| | 2-Hydroxy-2-sulfinatoessigsäure, Dinatriumsalz entsprechend Beispiel 3 | 79,5 | 82,5 | 1,02 | 0,34 |
| B | 0,3 | | | | |
| | Natriumformaldehydsulfoxylat | 79,5 | 79,7 | 1,34 | 0,46 |

| | | | | | |
|---|---|---|---|---|---|
| * Es wurde Natriumpyrophosphat als Komplexbildner zugesetzt | | | | | |

Die Zubereitung mit 2-Hydroxy-2-sulfinatoessigsäure, Dinatriumsalz zeigt gute Ergebnisse in der Kaolinbleiche. Im Vergleich zu Natriumformaldehydsulfoxylat reagierte die Zubereitung mit Hydroxyacetylsulfinsäure, Dinatriumsalz 3-4 mal schneller. Der Einsatz in der Mineralbleiche - insbesondere bei Kaolin - ist nach der gängigen Technologie möglich.

### Beispiel 7 (Vergleichsbeispiel)

400 g Wasser, 286 g einer 10%-igen wäßrigen Lösung von Airvol 205 (Polyvinylalkohol zu 88 % hydrolysiert; DP = 500; hergestellt von Air Products and Chemicals, Inc.), 286 g einer 10%-igen wäßrigen Lösung von Airvol 107 (Polyvinylalkohol zu 98 % hydrolysiert; DP - 500; hergestellt von Air Products and Chemicals, Inc. ) und 47 g Igepal CO-887 (Nichtionisches Tensid, hergestellt von Rhone-Poulenc, Inc. ; 70 %-ige wäßrige Lösung von Igepal CO-880 enthält ca. 30 mol Ethylenoxid) wurden in einen 3,8 Liter fassenden Druckreaktor gegeben und mit 4,8 g einer 1%-igen wäßrigen Eisen(II)-sulfatlösung vermischt. Die Reaktionsmischung wurde mit 1,75 g einer 50%-igen Phosphorsäurelösung auf pH 3,3 eingestellt. Anschließend wurden 1710 g Vinylacetat-Monomer zudosiert. Die Reaktionsmischung wurde bei 900 U/min gerührt und auf 35°C erwärmt. Im Folgenden wurden 200 g gasförmiges Ethylen mit einem Druck bis zu 20,4 atm eingeleitet. Im Weiteren wurden 5,7 g einer 10%igen wäßrigen Lösung von Isoascorbinsäure ( pH - 4 ) folgender Zusammensetzung:
270 g Wasser
30 g Isoascorbinsäure
0,8 g 29%-ige Ammoniumhydroxidlösung, zugegeben. Die Polymerisation wurde mit insgesamt 10 g einer 0,65%igen wäßrigen Wasserstoffperoxidlösung folgender Zusammensetzung:
589 g deionisiertes Wasser
11,1 g 35%-iges Wasserstoffperoxid, initiiert.
Nach der Initiierung der Polymerisation wurden die verbleibenden 295,1 g der Amonium-Isoascorbat/Isoascorbinsäure-Lösung innerhalb von 4 Stunden zudosiert. Durch die Zugabe der restlichen 590,1 g 0,65%iger Wasserstoffperoxidlösung wurde die Polymerisation so gesteuert, daß sich die Reaktionsmischung während eines Zeitraumes von 1 Stunde von 35°C auf 55°C erwärmte und daß die Reaktionsmischung anschließend während 3 Stunden auf 55°C gehalten werden konnte. Nach der Gesamtpolymerisation von 4 Stunden betrug der Gehalt an freiem Vinylacetat-Monomer noch 1,5%.

Das Reaktionsgemisch wurde auf 35°C abgekühlt und zur Entgasung von überschüssigem Ethylen in einen drucklosen Reaktor überführt. Das in der Emulsion verbliebene freie Vinylacetatmonomer wurde durch die Zugabe von 20 g einer 10%-igen wäßrigen Isoascorbinsäurelösung und einer 3, 5%-igen Wasserstoffperoxidlösung nachpolymerisiert und dadurch auf einen Endgehalt unter 0,5% freies Vinylacetat-Monomer gedrückt. Der pH-Wert der Polymeremulsion wurde mit einer 14 %-igen wäßrigen Ammoniumhydroxidlösung auf den gewünschten pH-Wert (siehe Tabelle 3) eingestellt. Die physikalischen Eigenschaften der Polymeremulsion (Latex) sind in Tabelle 3 zusammengefaßt.

### Beispiel 8 (Vergleichsbeispiel)

Die Emulsionspolymerisation entsprechend Beispiel 7 wurde wiederholt, und statt der Amonium-Isoascorbat/Isoascorbinsaure wurde eine wäßrige Lösung, bestehend aus 270 g Wasser und 22,1 g Natriumformaldehydsulfoxylat, verwendet. Die Ergebnisse sind in Tabelle 3 zusammengefaßt.

### Beispiel 9 (Co-Katalysator in der Emulsionspolymerisation)

Die Emulsionpolymerisation entsprechend Beispiel 7 wurde wiederholt, und statt der Ammonium-Isoascorbat/Isoascorbinsäure wurde eine wäßrige Lösung, bestehend aus 270 g Wasser und 33 g Reduktionsmittel gemäß Beispiel 1 (Rohprodukt), verwendet. Die Ergebnisse sind in Tabelle 3 zusammenfaßt.

**Tabelle 3**

| Parameter der erhaltenen Latices | Beispiel 7 (Vergleich) | Beispiel 8 (Vergleich) | Beispiel 9 (Erfindung) |
|---|---|---|---|
| Aussehen | leicht gelblich | milchig weiß | milchig weiß |
| Feststoffgehalt [%] | 62,1 | 63,4 | 63,2 |
| pH-Wert | 6,5 | 6,0 | 6,2 |
| Viskosität [Pa·s] | 440 | 380 | 560 |
| (60 U/min; 25 °C) | | | |
| Tg (Polymer) [°C] | +5 | +4 | +7 |
| freies Formaldehyd [ppm] | - | 130 | - |

### Beispiel 10

Holzschliffbleiche

| Bedingungen bei der Holzschliffbleiche: | |
|---|---|
| Stoffdichte | 5,4 % |
| Bleichtemperatur | 75°C |
| Bleichmittelzugabe | 0,2 / 0,4 / 0,6 / 0,8 / 1,0 % Bleichmittel atro (bez. auf Trockengewicht) |
| Bleichdauer | 30 Minuten |

Zur Bleiche wurden jeweils 100 g Holzschliff in Polyethylen-Beutel eingewogen. Für die Bleichmittelzugabe wurden wäßrige Lösungen hergestellt (1 ml dieser Lösungen enthielt je 0,2 % Bleichmittel atro). Nach dem Zupipettieren der Bleichmittellösung wurden die Beutel sofort verknotet, die Durchmischung erfolgte durch Kneten der geschlossenen Beutel. Die Bleichtemperatur wurde mit einem Thermostat (Wasserbad) geregelt.

Nach der erforderlichen Bleichdauer wurde der Stoffbrei in Meßbecher Überführt und der pH-Wert nach der Bleiche gemessen. Anschließend wurde mit Leitungswasser auf 300 ml aufgefüllt und durch Umrühren der Stoffbrei homogenisiert. Die Blattbildung wurde mit Hilfe eines üblichen Nutschen-Blattbildners unter Verwendung des gesamten Stoffbreis durchgeführt. Die erhaltenen Blätter wurden im Blattbildner 12 Minuten lang vakuumgetrocknet.

Von allen gebildeten Blättern wurde mit einem Weißgradmeßgerät (Elrepho 2000 von Datacolor) der Weißgrad R457 bestimmt. Die Ergebnisse sind in Tabelle 4 zusammengefaßt.

**Tabelle 4**

| Bleichmittel | Bleichmittelmenge [%atro] | pH-Wert Anfang | pH-Wert Ende | Weiße | Weißgradgewinn¹⁾ |
|---|---|---|---|---|---|
| Natriumformaldehydsulfoxylat | 0,0 | 6,4 | 6,3 | 65,1 | - |
| | 0,2 | 6,4 | 6,2 | 66,6 | 1,5 |
| | 0,4 | 6,4 | 6,2 | 66,9 | 1,8 |
| | 0,6 | 6,4 | 6,2 | 67,0 | 1,9 |
| | 0,8 | 6,4 | 6,2 | 67,3 | 2,2 |
| | 1,0 | 6,4 | 6,2 | 67,7 | 2,6 |
| 2-Hydroxy2-sulfinatoessigsäure, Dinatriumsalz entsprechend Beispiel 1 | 0,0 | 6,5 | 6,4 | 65,7 | - |
| | 0,2 | 6,5 | 6,4 | 66,7 | 1,0 |
| | 0,4 | 6,5 | 6,5 | 67,2 | 1,5 |
| | 0,6 | 6,5 | 6,6 | 67,6 | 1,9 |
| | 0,8 | 6,5 | 6,6 | 68,0 | 2,3 |
| | 1,0 | 6,5 | 6,7 | 68,1 | 2,4 |

| | | | | | |
|---|---|---|---|---|---|
| 1) gegenüber unbehandeltem Holzschliff | | | | | |

### Beispiel 11

### Deinktstoffbleiche

| Bedingungen bei der Deinktstoffbleiche: | |
|---|---|
| Stoffdichte | 7,4 % |
| Bleichtemperatur | 75°C |
| Bleichmittelzugabe | 0, 2 / 0, 4 / 0, 6 / 0, 8 / 1, 0 % Bleichmittel atro |
| Bleichdauer | 60 Minuten |

Zur Bleiche wurden jeweils 70g Deinktstoff in Polyethylen-Beutel eingewogen. Für die Bleichmittelzugabe wurden wäßrige Lösungen hergestellt (1 ml dieser Lösungen enthielt je 0,2 % Bleichmittel atro). Nach dem Zupipettieren der Bleichmittellösung wurden die Beutel sofort verknotet, die Durchmischung erfolgte durch Kneten der geschlossenen Beutel. Die Bleichtemperatur wurde mit einem Thermostat (Wasserbad) geregelt.

Nach der erforderlichen Bleichdauer wurde der Stoffbrei in Meßbecher überführt und der pH-Wert nach der Bleiche gemessen. Anschließend wurde mit Leitungswasser auf 300 ml aufgefüllt und durch Umrühren der Stoffbrei homogenisiert. Die Blattbildung wurde mit Hilfe eines üblichen Nutschen-Blattbildners unter Verwendung des gesamten Stoffbreis durchgeführt. Die erhaltenen Blätter wurden im Blattbildner 15 Minuten lang vakuumgetrocknet.

Von allen gebildeten Blättern wurde mit einem Weißgradmeßgerät (Elrepho 2000 von Datacolor) der Weißgrad R457 bestimmt. Die Ergebnisse sind in Tabelle 5 zusammengefaßt.

**Tabelle 5**

| Bleichmittel | Bleichmittelmenge [%atro] | pH-Wert Anfang | pH-Wert Ende | Weiße | Weißgradgewinn¹⁾ |
|---|---|---|---|---|---|
| Natriumformaldehydsulfoxylat | 0,0 | 7,2 | 7,2 | 64,5 | - |
| | 0,2 | 7,2 | 7,2 | 65,9 | 1,4 |
| | 0,4 | 7,2 | 7,3 | 66,3 | 1,8 |
| | 0,6 | 7,2 | 7,3 | 66,9 | 2,4 |
| | 0,8 | 7,2 | 7,3 | 66,9 | 2,4 |
| | 1,0 | 7,2 | 7,4 | 67,0 | 2,5 |
| 2-Hydroxy-2-sulfinatoessigsäure, Dinatriumsalz entsprechend Beispiel 1 | 0,0 | 7,2 | 7,2 | 64,5 | - |
| | 0,2 | 7,2 | 7,4 | 64,9 | 0,4 |
| | 0,4 | 7,2 | 7,4 | 66,0 | 1,5 |
| | 0,6 | 7,2 | 7,4 | 66,2 | 1,7 |
| | 0,8 | 7,2 | 7,5 | 66,5 | 2,0 |
| | 1,0 | 7,2 | 7,5 | 66,3 | 1,8 |

| | | | | | |
|---|---|---|---|---|---|
| 1) gegenüber unbehandeltem Deinktstoff | | | | | |

## Patentansprüche

1. Sulfinsäureverbindungen der allgemeinen Formel (I) 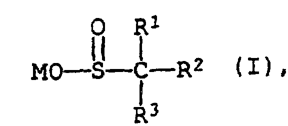 worin
M für ein Wasserstoffatom, ein Ammoniumion, ein einwertiges Metallion oder ein Äquivalent eines zweiwertigen Metallions der Gruppen Ia, IIa, IIb, IVa oder VIIIb des Periodensystems steht;
R¹ für OH oder NR⁴R⁵ steht, wobei R⁴ und R⁵ unabhängig voneinan der für H oder C₁-C₆-Alkyl stehen;
R² für H oder eine Alkyl-, Alkenyl-, Cycloalkyl- oder Aryl gruppe steht, wobei diese Gruppe 1, 2 oder 3 Substituenten aufweisen kann, die unabhängig voneinander ausgewählt sind unter C₁-C₆Alkyl, OH, O-C₁-C₆-Alkyl, Halogen und CF₃; und
R³ für COOM, SO₃M, COR⁴, CONR⁴R⁵ oder COOR⁴ steht, wobei M, R⁴ und R⁵ die oben angegebenen Bedeutungen besitzen,
und die Salze davon.

2. Sulfinsäureverbindungen nach Anspruch 1 der Formel (I),
worin
M für ein Ammonium- oder Alkalimetallion oder ein Äquivalent eines Erdalkalimetall- oder Zinkions steht.

3. Sulfinsäureverbindungen nach Anspruch 1 oder 2 der Formel (I),
worin
R¹ für OH oder NH₂ steht.

4. Sulfinsäureverbindungen nach Anspruch 1 der Formel (I),
worin
R² für ein Wasserstoffatom oder eine Alkyl- oder Arylgruppe steht, die einen oder zwei Hydroxy- oder Alkoxysubstituenten aufweisen kann.

5. Sulfinsäureverbindungen nach Anspruch 1 der Formel (I),
worin
R³ für COOM oder COOR⁴ steht, wobei M und R⁴ die in Anspruch 1 angegebenen Bedeutungen besitzen.

6. Sulfinsäureverbindungen nach Anspruch 1 der Formel (I),
worin
M für ein Alkalimetallion oder ein Äquivalent eines Erdalkalime tall- oder Zinkions steht;
R¹ für OH oder NH₂ steht;
R² für H oder Alkyl steht; und
R³ für COOM oder COOR⁴ steht, wobei M die angegebenen Bedeutungen besitzt und R⁴ für H oder C₁-C₆-Alkyl steht.

7. Verbindungen der Formeln (M = Na, K, Mg, Ca, Zn):

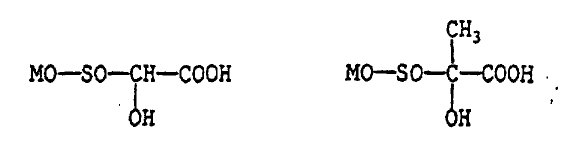

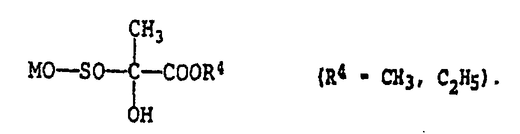

8. Gemisch einer Sulfinsäureverbindung nach einem der Ansprüche 1 7 mit der der Sulfinsäureverbindung entsprechenden Sulfonsäure bzw. dem Salz davon und gegebenenfalls mit dem entsprechenden Sulfit.

9. Gemisch nach Anspruch 8 folgender Zusammensetzung:
| | |
|---|---|
| Verbindung der Formel (I) | 20-99 Gew.-% |
| Sulfonsäure entsprechend der Verbindung der Formel (I) | bis zu 60 Gew.-% |
| M₂SO₃ | 0-40 Gew.-% |

10. Gemisch nach Anspruch 9 folgender Zusammensetzung:
| | |
|---|---|
| 2-Hydroxy-2-sulfinatoessigsäure, Dinatriumsalz | 40-73 Gew.-% |
| 2-Hydroxy-2-sulfonatoessigsaure, Dinatriumsalz | 2-7 Gew.-% |
| Natriumsulfit | 0-33 Gew.-% |
| Wasser | 5-30 Gew.-% |

11. Gemisch nach Anspruch 9 folgender Zusammensetzung:
| | |
|---|---|
| 2-Hydroxy-2-sulfinatoessigsäure, Zinksalz | 20-70 Gew.-% |
| 2-Hydroxy-2-sulfonatoessigsäure, Zinksalz | 5-60 Gew.-% |
| Wasser | 5-30 Gew.-%. |

12. Gemisch nach Anspruch 9 folgender Zusammensetzung:
| | |
|---|---|
| 2-Hydroxy-2-sulfinatopropionsäure, Dinatriumsalz | 38-70Gew.-% |
| 2-Hydroxy-2-sulfonatopropionsäure, Dinatriumsalz | 5-30Gew.-% |
| Natriumsulfit | 0-33Gew.-% |
| Wasser | 5-30 Gew.-%. |

13. Gemisch nach Anspruch 9 folgender Zusammensetzung:
| | |
|---|---|
| 2-Hydroxy-2-sulfinatopropionsäureethylester, Natriumsalz | 60-80 Gew.-% |
| 2-Hydroxy-2-sulfonatopropionsäureethylester, Natriumsalz | 0-5 Gew.-% |
| Natriumsulfit | 0-5 Gew.-% |
| Wasser | 5-20 Gew.-%. |

14. Zusammensetzung, enthaltend wenigstens eine Sulfinsäureverbindung nach einem der Ansprüche 1 - 7 oder wenigstens ein Gemisch nach einem der Ansprüche 8 - 13, zusammen mit üblichen Zusätzen und Hilfsstoffen.

15. Verwendung der Sulfinsäureverbindungen nach einem der Ansprüche 1-7 als Reduktionsmittel.

16. Verwendung nach Anspruch 15 als Reduktionsmittelkomponente für den Textildruck, in der Textilbleiche oder Küpenfärbung oder als reduktives Bleichmittel für die Mineralveredelung oder Faserveredelung.

17. Verwendung nach Anspruch 15 als Co-Katalysator in der Emulsionspolymerisation oder Redoxkatalysatorsystem bei der Kunststoffherstellung.

## Claims

1. Sulphinic acid compounds of general formula (I) 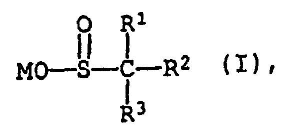 wherein
M denotes a hydrogen atom, an ammonium ion, a monovalent metal ion or an equivalent of a divalent metal ion of the groups Ia, IIa, IIb, IVa or VIIIb of the Periodic Table of Elements;
R¹ denotes OH or NR⁴R⁵, where R⁴ and R⁵ independently of each other denote H or C₁₋₆-alkyl;
R² denotes H or an alkyl, alkenyl, cycloalkyl or aryl group, where these groups may have 1, 2 or 3 substituents which are chosen independently of one another from C₁₋₆-alkyl, OH, O-C₁₋₆-alkyl, halogen and CF₃; and
R³ denotes COOM, SO₃M, COR⁴, CONR⁴R⁵ or COOR⁴, where M, R⁴ and R⁵ are defined as above,
and the salts thereof.

2. Sulphinic acid compounds according to claim 1 of formula (I), wherein
M denotes an ammonium or alkali metal ion or one equivalent of an alkaline earth metal ion or zinc ion.

3. Sulphinic acid compounds according to claim 1 or 2 of formula (I), wherein
R¹ denotes OH or NH₂.

4. Sulphinic acid compounds according to claim 1 of formula (I), wherein
R² is a hydrogen atom or an alkyl or aryl group which may have one or two hydroxyl or alkoxy substituents.

5. Sulphinic acid compounds according to claim 1 of formula (I), wherein
R³ denotes COOM or COOR⁴, where M and R⁴ are defined as in claim 1.

6. Sulphinic acid compounds according to claim 1 of formula (I), wherein
M denotes an alkali metal ion or one equivalent of an alkaline earth metal ion or zinc ion;
R¹ denotes OH or NH₂;
R² denotes H or alkyl; and
R³ denotes COOM or COOR⁴, where M has the meanings given and R⁴ denotes H or C₁₋₆-alkyl.

7. Compounds of the formulae (M = Na, K, Mg, Ca, Zn) :

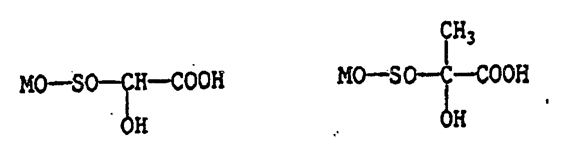

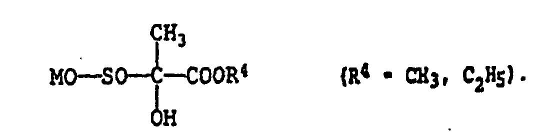

8. Mixture of a sulphinic acid compound according to one of claims 1 to 7 with the sulphonic acid corresponding to the sulphinic acid compound, or the salt thereof, and optionally with the corresponding sulphite.

9. Mixture according to claim 8 having the following composition:
| | |
|---|---|
| compound of formula (I) | 20-99 wt.% |
| sulphonic acid corresponding to the compound of formula (I) | up to 60 wt.% |
| M₂SO₃ | 0 - 40 wt .% |

10. Mixture according to claim 9 having the following composition:
| | |
|---|---|
| 2-hydroxy-2-sulphinatoacetic acid, disodium salt | 40-73 wt.% |
| 2-hydroxy-2-sulphonatoacetic acid, disodium salt | 2-7 wt.% |
| sodium sulphite | 0-33 wt.% |
| water | 5-30 wt.%. |

11. Mixture according to claim 9 having the following composition:
| | |
|---|---|
| 2-hydroxy-2-sulphinatoacetic acid, zinc salt | 20-70 wt.% |
| 2-hydroxy-2-sulphonatoacetic acid, zinc salt | 5-60 wt.% |
| water | 5-30 wt.%. |

12. Mixture according to claim 9 having the following composition:
| | |
|---|---|
| 2-hydroxy-2-sulphinatopropionic acid, disodium salt | 38-70 wt.% |
| 2-hydroxy-2-sulphonatopropionic acid, disodium salt | 5-30 wt.% |
| sodium sulphite | 0-33 wt.% |
| water | 5-30 wt.%. |

13. Mixture according to claim 9 having the following composition:
| | |
|---|---|
| ethyl 2-hydroxy-2-sulphinatopropionate, sodium salt | 60-80 wt.% |
| ethyl 2-hydroxy-2-sulphonatopropionate, sodium salt | 0-5 wt.% |
| sodium sulphite | 0-5 wt.% |
| water | 5-20 wt.%. |

14. Composition containing at least one sulphinic acid compound according to one of claims 1 to 7 or at least one mixture according to one of claims 8 to 13, together with conventional additives and adjuvants.

15. Use of the sulphinic acid compounds according to one of claims 1 to 7 as reducing agents.

16. Use according to claim 15 as reducing agent components for textile printing, in textile bleaching or vat dyeing or as a reductive bleaching agent for mineral or fibre finishing.

17. Use according to claim 15 as a co-catalyst in emulsion polymerisation or as a redox catalyst system in plastics manufacture.

## Revendications

1. Composés d'acide sulfinique de formule générale (I) : 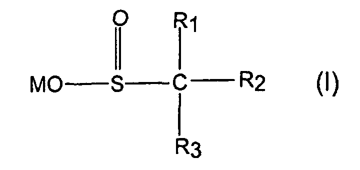 formule dans laquelle :
M représente un atome d'hydrogène, un ion ammonium, un ion métallique monovalent ou un équivalent d'un ion métallique bivalent des groupes Ia, IIa, IIb, IVa ou VIIIb du système périodique ;
R¹ représente le radical OH ou NR⁴R⁵, où R⁴ et R⁵ représentent indépendamment l'un de l'autre, l'atome d'hydrogène ou un radical alkyle en C₁-C₆ ;
R² représente l'atome d'hydrogène ou un radical alkyle, alcényle, cycloalkyle ou aryle, où ces radicaux peuvent présenter 1, 2 ou 3 substituants, qui sont choisis indépendamment l'un de l'autre parmi les radicaux alkyle en C₁-C₆, OH, O-alkyle en C₁-C₆,, halogène et CF₃, et
R³ représente les radicaux COOM, SO₃M, COR⁴, CONR⁴R⁵ ou COOR⁴, où M, R⁴ et R⁵ possèdent les significations indiquées ci-dessus,
et leurs sels.

2. Composés d'acide sulfinique selon la revendication 1 de formule (I), dans laquelle M représente un ion ammonium ou de métal alcalin ou un équivalent d'un ion de métal alcalinoterreux ou zinc.

3. Composés d'acide sulfinique selon la revendication 1 ou 2 de formule (I), dans laquelle R¹ représente OH ou NH₂.

4. Composés d'acide sulfinique selon la revendication 1 de formule (I), dans laquelle R² représente l'atome d'hydrogène ou un radical alkyle ou aryle, qui peut présenter un ou deux substituants hydroxy ou alcoxy.

5. Composés d'acide sulfinique selon la revendication 1 de formule (I), dans laquelle R³ représente COOM ou COOR⁴, où M et R⁴ possèdent les significations données à la revendication 1.

6. Composés d'acide sulfinique selon la revendication 1 de formule (I), dans laquelle
M représente un ion de métal alcalin ou un équivalent d'un ion de métal alcalinoterreux ou zinc ;
R¹ représente OH ou NH₂ ;
R² représente H ou un radical alkyle, et
R³ représente COOM ou COOR⁴, où M possède les significations indiquées et R⁴ représente H ou alkyle en C₁-C₆.

7. Composés des formules (M = Na, K, Mg, Ca, Zn) :

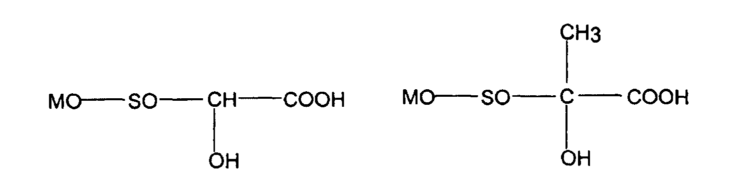

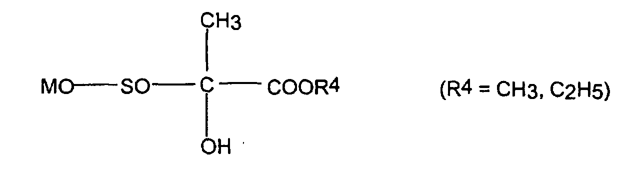

8. Mélange d'un composé d'acide sulfinique selon l'une quelconque des revendications 1 à 7, avec l'acide sulfonique correspondant à l'acide sulfinique ou son sel et, éventuellement, avec le sulfite correspondant.

9. Mélange selon la revendication 8, ayant la composition suivante :
| | |
|---|---|
| Composé de formule (I) | 20-99% en poids |
| Acide sulfonique correspondant au composé de formule (I) | jusqu'à 60% en poids |
| M₂SO₃ | 0-40% en poids |

10. Mélange selon la revendication 9, ayant la composition suivante :
| | |
|---|---|
| Sel disodique de l'acide 2-hydroxy-2-sulfinatoacétique | 40-73% en poids |
| Sel disodique de l'acide 2-hydroxy-2-sulfonatoacétique | 2-7% en poids |
| Sulfite de sodium | 0-33% en poids |
| Eau | 5-30% en poids |

11. Mélange selon la revendication 9, ayant la composition suivante :
| | |
|---|---|
| Sel de zinc de l'acide 2-hydroxy-2-sulfinatoacétique | 20-70% en poids |
| Sel de zinc de l'acide 2-hydroxy-2-sulfonatoacétique | 5-60% en poids |
| Eau | 5-30% en poids |

12. Mélange selon la revendication 9, ayant la composition suivante :
| | |
|---|---|
| Sel disodique de l'acide 2-hydroxy-2-sulfinatopropionique | 38-70% en poids |
| Sel disodique de l'acide 2-hydroxy-2-sulfonatopropionique | 5-30% en poids |
| Sulfite de sodium | 0-33% en poids |
| Eau | 5-30% en poids |

13. Mélange selon la revendication 9, ayant la composition suivante :
| | |
|---|---|
| Sel sodique de l'ester éthylique de l'acide 2-hydroxy-2-sulfinatopropionique | 60-80% en poids |
| Sel sodique de l'ester éthylique de l'acide 2-hydroxy-2-sulfonatopropionique | 0-5% en poids |
| Sulfite de sodium | 0-5% en poids |
| Eau | 5-20% en poids |

14. Composition contenant au moins un composé d'acide sulfinique selon l'une quelconque des revendications 1 à 7 ou au moins un mélange selon l'une quelconque des revendications 8 à 13, avec les additifs et auxiliaires usuels.

15. Utilisation des composés d'acide sulfinique selon l'une quelconque des revendications 1 à 7 comme agent de réduction.

16. Utilisation selon la revendication 15, comme composant d'un agent de réduction pour l'impression textile, le blanchiment des textiles ou la teinture en cuve ou comme agent de blanchiment réducteur pour l'amélioration de minéraux ou l'amélioration de fibres.

17. Utilisation selon la revendication 15, comme cocatalyseur dans la polymérisation en émulsion ou système de catalyse rédox dans la préparation des matières plastiques.
